(19) Europäisches Patentamt / European Patent Office / Office européen des brevets

(11) **EP 4 371 553 A1**

(12) **EUROPEAN PATENT APPLICATION**

(43) Date of publication:
**22.05.2024 Bulletin 2024/21**

(21) Application number: **22207767.9**

(22) Date of filing: **16.11.2022**

(51) International Patent Classification (IPC):
***A61K 9/00*** *(2006.01)* ***A61K 9/08*** *(2006.01)*
***A61K 47/26*** *(2006.01)* ***A61K 31/00*** *(2006.01)*
***A61K 47/10*** *(2017.01)*

(52) Cooperative Patent Classification (CPC):
**A61K 9/0019; A61K 9/08; A61K 31/58; A61K 47/10; A61K 47/26**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **B. Braun Melsungen AG**
**34212 Melsungen (DE)**

(72) Inventors:
- **FISCHER, Oliver**
  **34212 Melsungen (DE)**
- **SCHWEBEL, Herve**
  **34212 Melsungen (DE)**
- **ADAMO, Vincent**
  **34212 Melsungen (DE)**

(74) Representative: **Maiwald GmbH**
**Elisenhof**
**Elisenstraße 3**
**80335 München (DE)**

(54) **COMPOSITION COMPRISING ROCURONIUM**

(57) The present invention relates to a pharmaceutical composition comprising a pharmaceutically acceptable salt of rocuronium, at least one stabilizer, optionally a tonicity agent, and water, as well as to a method of manufacturing said pharmaceutical composition, and a container comprising said pharmaceutical composition.



Processed by Luminess, 75001 PARIS (FR)

## Description

### Field of the invention

The present invention relates to a pharmaceutical composition comprising a pharmaceutically acceptable salt of rocuronium, at least one stabilizer, optionally a tonicity agent, and water, as well as to a method of manufacturing said pharmaceutical composition, and a container comprising said pharmaceutical composition.

### Background

Anesthesia is typically defined as the elimination of certain body functions of a patient so that diagnostic or surgical procedures can be tolerated. Traditionally, anesthesia comprises the components of pain relief (analgesia), loss of consciousness (hypnosis), loss of vegetative functions and muscle relaxation (paralysis). These effects can be obtained from a single drug which alone provides the desired combination of effects, or a combination of drugs (such as hypnotics, sedatives, paralytics and analgesics) to achieve very specific combinations of effects.

Most currently available neuromuscular blocking agents comprise a quaternary ammonium structure, i.e. have a cationic scaffold. Such a structure allows for binding to the postsynaptic nicotinic acetylcholine receptor, thereby inhibiting or interfering with the binding of acetylcholine to the receptor finally leading to muscle relaxation.

One example of such compound is rocuronium which is an amino-steroid non-depolarizing neuromuscular blocker used in modern anesthesia to facilitate tracheal intubation through skeletal muscle relaxation.

Typically, such neuromuscular blocking agents are applied by intravenous injection. Usually, this requires dissolving said neuromuscular blocking agent that is provided, e.g. in the form of a freeze-dried powder containing the active ingredient and excipients, in a solvent containing water for injection and optionally co-solvents. However, such procedure does not only impose medical professionals with a high effort of preparing an injectable formulation but also bears the risk of medication errors as a consequence of wrong dilution.

Therefore, it is advantageous to provide neuromuscular blocking agents, such as rocuronium, in the form of ready-to-inject pharmaceutical formulations that are ready-to-use products which contain the active agent in dissolved form.

However, such pre-diluted products comprising the pharmaceutical compounds frequently suffer from high chemical instability of the active ingredient. Therefore, such formulations have a limited shelf live, but require cool chain shipment and storage. This is highly inconvenient and costly.

Specifically, pharmaceutically acceptable salts of rocuronium are prone to hydrolysis of the acetate ester contained in the molecule. This is particularly true when the pharmaceutically acceptable salt of rocuronium is stored in water, especially in the presence of acids or bases which are commonly known to catalyze or promote ester hydrolysis reactions.

Therefore, there is still a need for formulations comprising neuromuscular blocking agents, such as rocuronium, which are stable at room temperature and provide acceptable shelf life.

### Summary

The inventors of the present disclosure have surprisingly found that room temperature stable compositions of pharmaceutically acceptable salts of rocuronium can be prepared by using at least one stabilizing agent. When such formulation is placed in a container, the pharmaceutical composition remains stable, i.e. the amount of the pharmaceutically acceptable salt of rocuronium does not or substantially not decrease over time, even though the pharmaceutically acceptable salt of rocuronium is dissolved in water.

According to a first aspect, the present disclosure relates to a pharmaceutical composition comprising

a) a pharmaceutically acceptable salt of rocuronium,
b) at least one stabilizer,
c) optionally a tonicity agent, and
d) water,

wherein the at least one stabilizer is a polyol, and wherein the polyol does not comprise a -COOX group, wherein X is hydrogen or a pharmaceutically acceptable cation.

According to a second aspect, the present disclosure relates to a method for manufacturing the pharmaceutical composition according to the first aspect of the present disclosure comprising the steps of

e) dissolving the pharmaceutically acceptable salt of rocuronium in water and adjusting the pH with a strong acid

to provide a solution I;
f) dissolving the at least one stabilizer in water and adjusting the pH with a strong acid to provide a solution II;
g) mixing said solution I and said solution II and optionally adjusting the pH with a strong acid or a strong base to provide a solution III;
h) filling said solution III into a container and sealing said container; and
i) optionally sterilizing said container comprising solution III.

According to a third aspect, the present disclosure relates to another method for manufacturing the pharmaceutical composition according to the first aspect of the present disclosure comprising the steps of

a) dissolving the at least one stabilizer in water;
b) dissolving the pharmaceutically acceptable salt of rocuronium in said solution and optionally adjusting the pH with a strong acid or strong base;
c) filtering the solution;
d) filling said solution into a container and sealing said container; and
e) optionally sterilizing said container comprising the solution.

According to a fourth aspect, the present disclosure relates to a container comprising the pharmaceutical composition according to the first aspect of the present disclosure, wherein the container is a glass vial, a glass ampoule, a plastic ampoule, a plastic vial, a prefilled syringe, or an IV bag.

## Detailed Description

The invention of the present disclosure is described in the following in more detail, exemplified by preferred embodiments and embodiment examples. However, it is understood that the scope of the present disclosure is not limited thereto, but only by the appendant claims.

The present disclosure, in very general terms, relates to four aspects, namely a first aspect being directed to a pharmaceutical composition, a second and a third aspect being directed to a method for manufacturing said pharmaceutical composition, and a fourth aspect being directed to a container comprising said pharmaceutical composition.

### The pharmaceutical composition

According to a first aspect, the present disclosure relates to a pharmaceutical composition comprising

a) a pharmaceutically acceptable salt of rocuronium,
b) at least one stabilizer,
c) optionally a tonicity agent, and
d) water,

wherein the at least on stabilizer is a polyol, and wherein the polyol does not comprise a -COOX group, wherein X is hydrogen or a pharmaceutically acceptable cation.

#### *The polyol*

A core element of the pharmaceutical composition according to the present disclosure is the use of a specific stabilizer in connection with a pharmaceutically acceptable salt of rocuronium. Said stabilizer is a polyol, wherein the polyol does not comprise a -COOX group, wherein X is hydrogen or a pharmaceutically acceptable cation. In other words, the polyol is an organic polyol with at least two hydroxyl groups, but without an organic carboxylic acid or acid salt group.

According to a preferred embodiment of the present disclosure, the polyol is a sugar alcohol, or a monosaccharide, or a disaccharide, or a polysaccharide.

The inventors of the present disclosure have surprisingly found that polyol compounds, i.e. compounds comprising -CH(-OH)-groups and optionally -(C=O)-groups (wherein both groups are divalent groups), but do not contain -COOX groups or derivatives thereof, wherein X is hydrogen or a pharmaceutically acceptable cation, stabilize pharmaceutical compositions in liquid formulations. It is understood that, as terminal groups, the above given -CH(-OH)-group can be a -$CH_2$(-OH)-group, and the -(C=O)-group can be a -(CH(=O))-group. As an example, the compounds may comprise -$CH_2$ and -$CH_3$-groups.

In a preferred embodiment, the polyol compounds consist of -CH(-OH)-groups and optionally -(C=O)-groups

(wherein both groups are divalent groups), but do not contain -COOX groups or derivatives thereof. These compounds are composed of building blocks selected from -CH(-OH)-, $-CH_2(-OH)$, -(C=O)-, and -(CH(=O)), wherein at least two building blocks comprising a hydroxyl group are present. Carbonyl groups are optional, i.e., they may be present in the polyol compound.

It was surprisingly found that these polyols reduce the speed of decomposition of pharmaceutically acceptable rocuronium salts, in particular the speed of ester hydrolysis, in aqueous formulations. Therefore, said polyols have been found to surprisingly inhibit the acid catalysed or base mediated ester hydrolysis as is evidenced in Example 3 of the present disclosure.

According to another preferred embodiment of the present disclosure, the polyol is a sugar alcohol of formula I

$$HO\text{-}CH_2\text{-}[CH(OH)]_n\text{-}CH_2OH \qquad (I)$$

, wherein n is an integer of from 1 to 10. According to another preferred embodiment of the present disclosure, polyol is a sugar alcohol of the formula I, wherein n is an integer of from 2 to 6. According to another preferred embodiment of the present disclosure, the polyol is a sugar alcohol of formula I, wherein n is an integer of from 3 to 5. According to a further preferred embodiment of the present disclosure, the polyol is a sugar alcohol of formula I wherein n is 4.

According to another further preferred embodiment of the present disclosure, the polyol is a sugar alcohol selected from the group consisting of glucose, glycerol, sorbitol, erythritol, threitol, arabitol, xylitol, ribitol, galactitol, fucitol, iditol, inositol, volemitol, isomalt, maltitol, and lactitol.

According to another preferred embodiment of the present disclosure, the at least one stabilizer is mannitol, glycerol, or sorbitol. According to a further preferred embodiment of the present disclosure, the at least one stabilizer is mannitol. According to another further preferred embodiment of the present disclosure, the at least one stabilizer is glycerol. According to another further preferred embodiment of the present disclosure, the at least one stabilizer is sorbitol. According to another further preferred embodiment of the present disclosure, the at least one stabilizer is xylitol.

The inventors of the present disclosure have surprisingly found that sugar alcohols, and specifically mannitol, glycerol, sorbitol, and/or xylitol, significantly stabilize solutions containing pharmaceutically acceptable salts of rocuronium, as evidenced in Examples 1 to 3 of the present disclosure. In this context, it is noted that the stability of rocuronium formulations comprising sugar alcohols of formula I according to the present disclosure is superior to the formulations according to the prior art as evidenced in Examples 1 and 2.

According to another preferred embodiment of the present disclosure, the polyol is a monosaccharide of the formula II

$$HO\text{-}CH_2\text{-}[CH(OH)]_n\text{-}(C{=}O)\text{-}[CH(OH)]_m\text{-}H \qquad (II)$$

, or an intramolecular lactole thereof, wherein n is an integer of from 1 to 10, and wherein m is an integer of from 0 to 2. According to another preferred embodiment of the present disclosure, the polyol is a monosaccharide of the formula II, wherein n is an integer in the range of from 2 to 6, and wherein m is an integer on the range of from 0 to 2. According to another preferred embodiment of the present disclosure, the polyol is a monosaccharide of the formula II, wherein n is 3 or 4, and wherein m is an integer on the range of from 0 to 2.

According to a further preferred embodiment of the present disclosure, the polyol is a monosaccharide of the formula II, wherein n is an integer in the range of from 2 to 6, and wherein m is 0 or 1. According to a further preferred embodiment of the present disclosure, the polyol is a monosaccharide of the formula II, wherein n is 3 or 4, and wherein m is 0 or 1.

According to another preferred embodiment of the present disclosure, the polyol is a monosaccharide of formula II, wherein the number of carbon atoms x is an integer in the range of from 3 to 14. According to another preferred embodiment of the present disclosure, the polyol is a monosaccharide of formula II, wherein the number of carbon atoms x is an integer in the range of from 3 to 10. According to another preferred embodiment of the present disclosure, the polyol is a monosaccharide of formula II, wherein the number of carbon atoms x is an integer in the range of from 3 to 8. According to another preferred embodiment of the present disclosure, the polyol is a monosaccharide of formula II, wherein the number of carbon atoms x is an integer in the range of from 3 to 7.

According to another preferred embodiment of the present disclosure, the polyol is a monosaccharide of formula II, wherein the number of carbon atoms x is 3. In other words, the polyol is a monosaccharide of formula II, wherein n is 1 and m is 0.

According to another preferred embodiment of the present disclosure, the polyol is a monosaccharide of formula II, wherein the number of carbon atoms x is 4. In other words, the polyol is a monosaccharide of formula II, wherein n is 2 and m is 0, or wherein n is 1 and m is 1.

According to another preferred embodiment of the present disclosure, the polyol is a monosaccharide of formula II, wherein the number of carbon atoms x is 7. In other words, the polyol is a monosaccharide of formula II, wherein n

is 5 and m is 0, or wherein n is 4 and m is 1, or wherein n is 3 and m is 2.

According to another preferred embodiment of the present disclosure, the polyol is a monosaccharide of formula II, wherein the number of carbon atoms x is 8. In other words, the polyol is a monosaccharide of formula II, wherein n is 6 and m is 0, or wherein n is 5 and m is 1, or wherein n is 4 and m is 2.

According to a further preferred embodiment of the present disclosure, the polyol is a monosaccharide of formula II, wherein the number of carbon atoms x is 5. In other words, the polyol is a monosaccharide of formula II, wherein n is 3 and m is 0, or wherein n is 2 and m is 1, or wherein n is 1 and m is 2. According to another further preferred embodiment of the present disclosure, the polyol is selected from the group consisting of arabinose, lyxose, ribose, xylose, arabinose, ribulose, and xylolose.

According to a further preferred embodiment of the present disclosure, the polyol is monosaccharide of the formula II, wherein the number of carbon atoms x is 6. In other words, the polyol is a monosaccharide of formula II, wherein n is 4 and m is 0, or wherein n is 3 and m is 1, or wherein n is 2 and m is 2. According to a further preferred embodiment of the present disclosure, the polyol is selected from the group consisting of allose, altrose, glucose, mannose, gulose, idose, galactose, talose, psicose, fructose, sorbose, tagatose, and glutose.

According to a preferred embodiment of the present disclosure, the polyol is selected from the group consisting of glyceraldehyde, erythrose, threose, erythrulose, arabinose, lyxose, ribose, xylose, arabinose, ribulose, xylolose, allose, altrose, glucose, mannose, gulose, idose, galactose, talose, psicose, fructose, sorbose, tagatose, glutose, sedoheptulose, mannoheptulose, and *glycerol-manno*-heptose.

According to a further preferred embodiment of the present disclosure, the polyol is selected from the group consisting of glucose, fructose, galactose, and mixtures thereof.

According to a further preferred embodiment of the present disclosure, the at least one stabilizer is selected from the group consisting of glucose, fructose, galactose, and mixtures thereof. According to a further preferred embodiment of the present disclosure, the at least one stabilizer is glucose. According to a further preferred embodiment of the present disclosure, the at least one stabilizer is fructose. According to a further preferred embodiment of the present disclosure, the at least one stabilizer is galactose.

As disclosed herein, it was surprisingly found that monosaccharides, and glucose in particular, stabilize pharmaceutically acceptable salts of rocuronium as evidenced in Example 3 of the present disclosure. In this context, it is noted that the stability of rocuronium formulations comprising a monosaccharide of formula I of the present disclosure is superior to the formulations according to the prior art as evidenced in Example 3 in contrast to Examples 1 and 2.

According to another preferred embodiment of the present disclosure, the polyol is a disaccharide. According to another preferred embodiment of the present disclosure, the polyol is a disaccharide selected from the group consisting of sucrose, lactose, maltose, trehalose, cellobiose, chitobiose, kojibiose, nigerose, isomaltose, sophorose, laminaribiose, gentiobiose, trehalulose, turanose, maltulose, leucrose, isomaltulose, gentiobiulose, mannobiose, melibiose, rutinose, rutinulose, and xylobiose. According to another preferred embodiment of the present disclosure, the polyol is a disaccharide selected from the group consisting of sucrose, maltose, maltulose, isomaltose, lactose, lactulose, trehalose, and mixtures thereof.

According to a further preferred embodiment of the present disclosure, the at least one stabilizer is sucrose. According to a further preferred embodiment of the present disclosure, the at least one stabilizer is maltose. According to a further preferred embodiment of the present disclosure, the at least one stabilizer is lactose. According to a further preferred embodiment of the present disclosure, the at least one stabilizer is trehalose.

The inventors of the present disclosure have surprisingly found that also disaccharides, and specifically sucrose, lactose, and trehalose, significantly stabilize pharmaceutically acceptable salts of rocuronium as evidenced in Example 3 of the present disclosure. In this context, it is noted that the stability of rocuronium formulations comprising sugar alcohols of formula I of the present disclosure is superior to the formulations according to the prior art as evidenced in Example 3 as opposed to formulations comprising carboxylic acids (Examples 1 and 2).

According to another preferred embodiment of the present disclosure, the polyol is a polysaccharide. According to another preferred embodiment of the present disclosure, the polysaccharide is a dextran. According to a further preferred embodiment of the present disclosure, the dextran is dextran 40. According to another preferred embodiment of the present disclosure, the polysaccharide is a HES compound. According to a further preferred embodiment of the present disclosure, the HES compound is HES130.

The inventors of the present disclosure have surprisingly found that also polysaccharides, and specifically dextran 40, and HES130 significantly stabilize pharmaceutically acceptable salts of rocuronium as evidenced in example 3 of the present disclosure. In this context, it is noted that the stability of rocuronium formulations comprising sugar alcohols of formula I of the present disclosure is superior to the formulations according to the prior art as evidenced in Example 3 as opposed to Examples 1 and 2.

*The stabilizer*

The pharmaceutical composition of the present disclosure comprises at least one stabilizer. According to a preferred embodiment of the present disclosure, the pharmaceutical composition comprises five stabilizers or less. In other words, according to a preferred embodiment of the present disclosure, the pharmaceutical composition comprises one, two, three, four, or five stabilizers. According to another preferred embodiment of the present disclosure, the pharmaceutical composition comprises four stabilizers or less. In other words, according to a preferred embodiment of the present disclosure, the pharmaceutical composition comprises one, two, three, or four stabilizers. According to another preferred embodiment of the present disclosure, the pharmaceutical composition comprises 3 stabilizers or less. In other words, according to a preferred embodiment of the present disclosure, the pharmaceutical composition comprises one, two, or three stabilizers. According to a further preferred embodiment of the present disclosure, the pharmaceutical composition comprises one or two stabilizers. According to a further preferred embodiment of the present disclosure, the pharmaceutical composition comprises one stabilizer.

If more than one stabilizer is used in the compositions of the present disclosure, the stabilizer may be independently selected from the above given stabilizers. As such, two or more stabilizers may be present as mixture of stabilizers, where each stabilizer is independently selected from the above given stabilizers, and in particular preferred embodiments of stabilizers, i.e., polyols, may be combined with each other. The combination of polyols may have a synergistic effect.

The inventors of the present disclosure have surprisingly found that pharmaceutical compositions of rocuronium salts do not require complicated stabilizing systems, i.e. a combination of several stabilizers. To the contrary, it is evidenced in Examples 1 to 3 that a pharmaceutical composition of rocuronium can be sufficiently stabilized by addition of only one stabilizer. Therefore, the effort of production is low.

According to a preferred embodiment of the present disclosure, the pharmaceutical composition comprises the at least one stabilizer in a concentration of about 250 mg/mL or less. According to a preferred embodiment of the present disclosure, the pharmaceutical composition comprises the at least one stabilizer in a concentration of about 250 mg/mL or less. According to another preferred embodiment of the present disclosure, the pharmaceutical composition comprises the at least one stabilizer in a concentration of about 200 mg/mL or less. According to another preferred embodiment of the present disclosure, the pharmaceutical composition comprises the at least one stabilizer in a concentration of about 180 mg/mL or less. According to another preferred embodiment of the present disclosure, the pharmaceutical composition comprises the at least one stabilizer in a concentration of about 150 mg/mL or less. According to another preferred embodiment of the present disclosure, the pharmaceutical composition comprises the at least one stabilizer in a concentration of about 140 mg/mL or less. According to another preferred embodiment of the present disclosure, the pharmaceutical composition comprises the at least one stabilizer in a concentration of about 130 mg/mL or less. According to another preferred embodiment of the present disclosure, the pharmaceutical composition comprises the at least one stabilizer in a concentration of about 120 mg/mL or less. According to another preferred embodiment of the present disclosure, the pharmaceutical composition comprises the at least one stabilizer in a concentration of about 110 mg/mL or less. According to a further preferred embodiment of the present disclosure, the pharmaceutical composition comprises the at least one stabilizer in a concentration of about 100 mg/mL or less.

According to another preferred embodiment of the present disclosure, the pharmaceutical composition comprises the at least one stabilizer in a concentration of about 1 mg/mL or more. According to another preferred embodiment of the present disclosure, the pharmaceutical composition comprises the at least one stabilizer in a concentration of about 3 mg/mL or more. According to a further preferred embodiment of the present disclosure, the pharmaceutical composition comprises the at least one stabilizer in a concentration of about 5 mg/mL or more. According to a further preferred embodiment of the present disclosure, the pharmaceutical composition comprises the at least one stabilizer in a concentration of about 10 mg/mL or more.

According to another preferred embodiment of the present disclosure, the pharmaceutical composition comprises the at least one stabilizer in a concentration in the range of from about 1 mg/mL to about 200 mg/mL. According to another preferred embodiment of the present disclosure, the pharmaceutical composition comprises the at least one stabilizer in a concentration in the range of from about 1 mg/mL to about 150 mg/mL. According to another preferred embodiment of the present disclosure, the pharmaceutical composition comprises the at least one stabilizer in a concentration in the range of from about 3 mg/mL to about 150 mg/mL. According to another preferred embodiment of the present disclosure, the pharmaceutical composition comprises the at least one stabilizer in a concentration in the range of from about 3 mg/mL to about 140 mg/mL. According to another preferred embodiment of the present disclosure, the pharmaceutical composition comprises the at least one stabilizer in a concentration in the range of from about 5 mg/mL to about 130 mg/mL. According to another preferred embodiment of the present disclosure, the pharmaceutical composition comprises the at least one stabilizer in a concentration in the range of from about 5 mg/mL to about 120 mg/mL. According to another preferred embodiment of the present disclosure, the pharmaceutical composition comprises the at least one stabilizer in a concentration in the range of from about 5 mg/mL to about 110 mg/mL. According to a further

preferred embodiment of the present disclosure, the pharmaceutical composition comprises the at least one stabilizer in a concentration in the range of from about 5 mg/mL to about 100 mg/mL. According to a further preferred embodiment of the present disclosure, the pharmaceutical composition comprises the at least one stabilizer in a concentration in the range of from about 10 mg/mL to about 100 mg/mL.

The inventors of the present disclosure have surprisingly found that the polyols according to the present disclosure are effective in preventing rocuronium from hydrolysing already at minimal concentrations as evidenced in Examples 1 to 3.

According to another preferred embodiment of the present disclosure, the pharmaceutical composition comprises the pharmaceutically acceptable salt of rocuronium in a concentration of from about 0.1 mg/mL to about 100 mg/mL. According to another preferred embodiment of the present disclosure, the pharmaceutical composition comprises the pharmaceutically acceptable salt of rocuronium in a concentration of from about 1 mg/mL to about 20 mg/mL. According to another preferred embodiment of the present disclosure, the pharmaceutical composition comprises the pharmaceutically acceptable salt of rocuronium in a concentration more preferably of from about 5 mg/mL to about 15 mg/mL. According to a further preferred embodiment of the present disclosure, the pharmaceutical composition comprises the pharmaceutically acceptable salt of rocuronium in a concentration of from about 8 mg/mL to about 12 mg/mL, most preferably about 10 mg/mL.

*Further ingredients*

According to a preferred embodiment of the present disclosure, the pharmaceutically acceptable salt of rocuronium is rocuronium bromide (CAS No. 119302-91-9).

According to another preferred embodiment of the present disclosure, the pharmaceutical composition comprises a tonicity agent. It is understood that a tonicity agent does not have a stabilizing effect, but is merely present in the pharmaceutical composition to adjust the tonicity of the solution. In other words, the pharmaceutical composition according to the first aspect of the present disclosure comprises both at least one stabilizer and a tonicity agent. According to a further preferred embodiment of the present disclosure, the tonicity agent is NaCl.

According to another preferred embodiment of the present disclosure, the pharmaceutical composition has a pH in the range of from about 3.0 to about 4.5. According to another preferred embodiment of the present disclosure, the pharmaceutical composition has a pH in the range of from about 3.2 to about 4.2. According to another preferred embodiment of the present disclosure, the pharmaceutical composition has a pH in the range of from about 3.0 to about 3.5. According to a further preferred embodiment of the present disclosure, the pharmaceutical composition has a pH in the range more preferably of from about 3.8 to about 4.2.

According to a preferred embodiment of the present disclosure, the pharmaceutical composition has an osmolarity of 600 mOsmol/L or less. According to another preferred embodiment of the present disclosure, the pharmaceutical composition has an osmolarity in the range of from about 32 mOsmol/L to about 600 mOsmol/L. According to another preferred embodiment of the present disclosure, the pharmaceutical composition has an osmolarity in the range of from about 100 mOsmol/L to about 600 mOsmol/L. According to another preferred embodiment of the present disclosure, the pharmaceutical composition has an osmolarity in the range of from about 120 mOsmol/L to about 500 mOsmol/L. According to another preferred embodiment of the present disclosure, the pharmaceutical composition has an osmolarity in the range of from about 150 mOsmol/L to about 450 mOsmol/L. According to another preferred embodiment of the present disclosure, the pharmaceutical composition has an osmolarity in the range of from about 200 mOsmol/L to about 400 mOsmol/L. According to another preferred embodiment of the present disclosure, the pharmaceutical composition has an osmolarity in the range of from about 230 mOsmol/L to about 380 mOsmol/L. According to another preferred embodiment of the present disclosure, the pharmaceutical composition has an osmolarity in the range of from about 250 mOsmol/L to about 350 mOsmol/L. According to a further preferred embodiment of the present disclosure, the pharmaceutical composition has an osmolarity in the range most preferably of from about 270 mOsmol/L to about 310 mOsmol/L.

According to a preferred embodiment of the present disclosure, the pharmaceutical composition does not contain EDTA, or a salt thereof, or a derivative thereof. According to another preferred embodiment of the present disclosure, the pharmaceutical composition does not contain glycine, or a salt thereof, or a derivative thereof. According to another preferred embodiment of the present disclosure, the pharmaceutical composition does not contain citric acid, or a salt thereof, or a derivative thereof. According to another preferred embodiment of the present disclosure, the pharmaceutical composition does not contain gluconic acid, or a salt thereof, or a derivative thereof. According to another preferred embodiment of the present disclosure, the pharmaceutical composition does not contain acetic acid, or a salt thereof, or a derivative thereof.

According to another preferred embodiment of the present disclosure, the pharmaceutical composition does not contain EDTA, or glycine, or citric acid, or acetic acid, or gluconic acid, or a salt thereof, or a derivative thereof. According to a further preferred embodiment of the present disclosure, the pharmaceutical composition does not contain

a weak acid or a salt thereof or a derivative thereof.

Without being bound to any theory, it is assumed or was shown in the Examples below that any of the above listed compounds which should preferably not be contained in the pharmaceutical composition of the present disclosure are detrimental to the stability of solutions containing rocuronium.

According to the present disclosure, the pharmaceutical composition is considered not to contain the compounds defined above (that are, EDTA, or a salt thereof, or a derivative thereof; glycine, or a salt thereof, or a derivative thereof; citric acid, or a salt thereof, or a derivative thereof; gluconic acid, or a salt thereof, or a derivative thereof acetic acid, or a salt thereof, or a derivative thereof; and or a weak acid, or a salt thereof or a derivative thereof), if the molar amount of said compound is lower than or equal to the molar amount of rocuronium or pharmaceutically acceptable salt thereof added during preparation. In other words, it is understood that the pharmaceutical composition does not contain EDTA, glycine, citric acid, gluconic acid, acetic acid, a weak acid, any salt thereof, and/or any derivative thereof in a molar amount that exceeds the molar amount of rocuronium or pharmaceutically acceptable salt thereof used for the preparation of said pharmaceutical composition. Such concentrations do not lead to a significant detrimental effect.

The inventors of the present invention have surprisingly found that pharmaceutical compositions comprising no weak acids, or salts thereof, or derivative thereof exhibit reduced hydrolysis and degradation of rocuronium. This finding is against the teaching of the prior art. This is, for instance, evidenced in Examples 1 and 2 of the present disclosure.

According to another preferred embodiment of the present disclosure, the content of a pharmaceutically acceptable salt of rocuronium, is at least 95 % (m/m) after storing the pharmaceutical composition for 18 months or more at room temperature based on the initial content of the pharmaceutically acceptable salt of rocuronium. According to another preferred embodiment of the present disclosure, the content of a pharmaceutically acceptable salt of rocuronium, is at least 96 % (m/m) after storing the pharmaceutical composition for 18 months or more at room temperature based on the initial content of the pharmaceutically acceptable salt of rocuronium. According to another preferred embodiment of the present disclosure, the content of a pharmaceutically acceptable salt of rocuronium, is at least 97 % (m/m) after storing the pharmaceutical composition for 18 months or more at room temperature based on the initial content of a pharmaceutically acceptable salt of rocuronium. According to a further preferred embodiment of the present disclosure, the content of a pharmaceutically acceptable salt of rocuronium, is at least 98 % (m/m) after storing the pharmaceutical composition for 18 months or more at room temperature based on the initial content of the pharmaceutically acceptable salt of rocuronium.

According to another preferred embodiment of the present disclosure, the content of a pharmaceutically acceptable salt of rocuronium, is at least 95 % (m/m) after storing the pharmaceutical composition for 17 days or more at 50 °C based on the initial content of a pharmaceutically acceptable salt of rocuronium. According to another preferred embodiment of the present disclosure, the content of a pharmaceutically acceptable salt of rocuronium, is at least 96 % (m/m) after storing the pharmaceutical composition for 17 days or more at 50 °C based on the initial content of a pharmaceutically acceptable salt of rocuronium. According to another preferred embodiment of the present disclosure, the content of a pharmaceutically acceptable salt of rocuronium, is at least 97 % (m/m) after storing the pharmaceutical composition for 17 days or more at 50 °C based on the initial content of the pharmaceutically acceptable salt of rocuronium. According to a further preferred embodiment of the present disclosure, the content of a pharmaceutically acceptable salt of rocuronium, is at least 98 % (m/m) after storing the pharmaceutical composition for 17 months or more at 50 °C based on the initial content of a pharmaceutically acceptable salt of rocuronium.

According to another preferred embodiment of the present disclosure, the content of a pharmaceutically acceptable salt of rocuronium, is at least 95 % (m/m) after storing the pharmaceutical composition for 17 days or more at 70 °C based on the initial content of a pharmaceutically acceptable salt of rocuronium. According to another preferred embodiment of the present disclosure, the content of a pharmaceutically acceptable salt of rocuronium, is at least 96 % (m/m) after storing the pharmaceutical composition for 17 days or more at 70 °C based on the initial content of a pharmaceutically acceptable salt of rocuronium. According to another preferred embodiment of the present disclosure, the content of a pharmaceutically acceptable salt of rocuronium, is at least 97 % (m/m) after storing the pharmaceutical composition for 17 days or more at 70 °C based on the initial content of the pharmaceutically acceptable salt of rocuronium.

According to another preferred embodiment of the present disclosure, the content of impurity C in the pharmaceutical composition is 5 % (m/m) or less after storing the pharmaceutical composition for 18 months or more at room temperature based on the initial content of the pharmaceutically acceptable salt of rocuronium. According to another preferred embodiment of the present disclosure, the content of impurity C in the pharmaceutical composition is 4 % (m/m) or less after storing the pharmaceutical composition for 18 months or more at room temperature based on the initial content of the pharmaceutically acceptable salt of rocuronium. According to another preferred embodiment of the present disclosure, the content of impurity C in the pharmaceutical composition is at 3 % (m/m) or less after storing the pharmaceutical composition for 18 months or more at room temperature based on the initial content of a pharmaceutically acceptable salt of rocuronium.

According to another preferred embodiment of the present disclosure, the content of impurity C in the pharmaceutical composition is 5 % (m/m) or less after storing the pharmaceutical composition for 17 days or more at 50 °C

based on the initial content of a pharmaceutically acceptable salt of rocuronium. According to another preferred embodiment of the present disclosure the content of impurity C in the pharmaceutical composition is at 4 % (m/m) or less after storing the pharmaceutical composition for 17 days or more at 50 °C based on the initial content of a pharmaceutically acceptable salt of rocuronium. According to another preferred embodiment of the present disclosure, the content of impurity C in the pharmaceutical composition is 2 % (m/m) or less after storing the pharmaceutical composition for 17 days or more at 50 °C based on the initial content of the pharmaceutically acceptable salt of rocuronium.

According to another preferred embodiment of the present disclosure the content of impurity C in the pharmaceutical composition is 5 % (m/m) or less after storing the pharmaceutical composition for 17 days or more at 70 °C based on the initial content of a pharmaceutically acceptable salt of rocuronium. According to another preferred embodiment of the present disclosure, the content of impurity C in the pharmaceutical composition is 4 % (m/m) or less after storing the pharmaceutical composition for 17 days or more at 70 °C based on the initial content of a pharmaceutically acceptable salt of rocuronium. According to another preferred embodiment of the present disclosure, the content of impurity C in the pharmaceutical composition is 3 % (m/m) or less after storing the pharmaceutical composition for 17 days or more at 70 °C based on the initial content of the pharmaceutically acceptable salt of rocuronium.

As evidenced in Example 3, the pharmaceutical compositions according to the present disclosure show superior stability in that the pharmaceutical composition is not or substantially not susceptible to hydrolysis.

<u>The method of manufacturing</u>

According to a second aspect, the present disclosure relates to a method for manufacturing the pharmaceutical composition according to the first aspect of the present disclosure comprising the steps of

a) dissolving the pharmaceutically acceptable salt of rocuronium in water and adjusting the pH with a strong acid to provide a solution I;
b) dissolving the at least one stabilizer in water and adjusting the pH with a strong acid to provide a solution II;
c) mixing said solution I and said solution II and optionally adjusting the pH with a strong acid or a strong base to provide a solution III;
d) filling said solution III into a container and sealing said container; and
e) optionally sterilizing said container comprising solution III.

According to a third aspect, the present disclosure relates to a method for manufacturing the pharmaceutical composition according to the first aspect of the present disclosure comprising the steps of

a) dissolving the at least one stabilizer in water;
b) dissolving the pharmaceutically acceptable salt of rocuronium in said solution and optionally adjusting the pH with a strong acid or strong base;
c) filtering the solution;
d) filling said solution into a container and sealing said container; and
e) optionally sterilizing said container comprising the solution.

According to a preferred embodiment of the present disclosure, the method further comprises adding a tonicity agent to solution I. According to a preferred embodiment of the present disclosure, the method further comprises adding a tonicity agent to solution II. According to a preferred embodiment of the present disclosure, the method further comprises adding a tonicity agent to solution III. According to a preferred embodiment of the present disclosure, the method further comprises adding a tonicity agent to solution I or solution II or solution III.

According to a preferred embodiment of the present disclosure, the method further comprises adding a tonicity agent to a solution.

According to another preferred embodiment of the present disclosure, step d) is carried out under aseptic conditions.

According to a preferred embodiment of the present disclosure, the sterilizing is performed by heat sterilization.

According to another preferred embodiment of the present disclosure, the sterilizing is performed by heat sterilization for a period $F_0$ of at least about 1 min. According to another preferred embodiment of the present disclosure, the sterilizing is performed by heat sterilization for a period $F_0$ of at least about 5 min. According to a further preferred embodiment of the present disclosure, the sterilizing is performed by heat sterilization for a period $F_0$ of at least about 8 min.

According to a preferred embodiment of the present disclosure, the sterilizing is performed by heat sterilization for a period $F_0$ of about 12 h or less. According to another preferred embodiment of the present disclosure, the sterilizing is performed by heat sterilization for a period $F_0$ of about 1 h or less. According to another preferred embodiment of the present disclosure, the sterilizing is performed by heat sterilization for a period $F_0$ of about 45 min or less. According to

another preferred embodiment of the present disclosure, the sterilizing is performed by heat sterilization for a period $F_0$ of about 30 min or less. According to a further preferred embodiment of the present disclosure, the sterilizing is performed by heat sterilization for a period $F_0$ of about 20 min or less.

According to a preferred embodiment of the present disclosure, the sterilizing is performed by heat sterilization for a period $F_0$ in the range of from about 1 min to about 20 h. According to another preferred embodiment of the present disclosure, the sterilizing is performed by heat sterilization for a period $F_0$ in the range of from about 5 min to about 20 h. According to another preferred embodiment of the present disclosure, the sterilizing is performed by heat sterilization for a period $F_0$ in the range of from about 8 min to about 20 h. According to another preferred embodiment of the present disclosure, the sterilizing is performed by heat sterilization for a period $F_0$ in the range of from about 5 min to about 12 h. According to another preferred embodiment of the present disclosure, the sterilizing is performed by heat sterilization for a period $F_0$ in the range of from about 5 min to about 1 h. According to another preferred embodiment of the present disclosure, the sterilizing is performed by heat sterilization for a period $F_0$ in the range of from about 5 min to about 45 min. According to another preferred embodiment of the present disclosure, the sterilizing is performed by heat sterilization for a period $F_0$ in the range of from about 5 min to about 30 min. According to another preferred embodiment of the present disclosure, the sterilizing is performed by heat sterilization for a period $F_0$ in the range of from about 5 min to about 20 min. According to another preferred embodiment of the present disclosure, the sterilizing is performed by heat sterilization for a period $F_0$ in the range of from about 8 min to about 1 h. According to another preferred embodiment of the present disclosure, the sterilizing is performed by heat sterilization for a period $F_0$ in the range of from about 8 min to about 45 min. According to another preferred embodiment of the present disclosure, the sterilizing is performed by heat sterilization for a period $F_0$ in the range of from about 8 min to about 30 min. According to a further embodiment of the present disclosure, the sterilizing is performed by heat sterilization for a period $F_0$ in the range of from about 8 min to about 20 min.

According to a preferred embodiment of the present disclosure, sterilizing is performed by heat sterilization at a temperature T of about 121 °C or more. According to a further preferred embodiment of the present disclosure, sterilizing is performed by heat sterilization at a temperature T of about 121 °C.

According to another preferred embodiment of the present disclosure, sterilizing is performed by heat sterilization at a temperature T of about 118 °C or less. According to a further preferred embodiment of the present disclosure, sterilizing is performed by heat sterilization at a temperature T of about 112 °C.

It is understood that the time for which sterilizing is performed is inextricably linked to the temperature T at which sterilizing is performed. In this context, it is understood that a higher temperature T, such as a temperature T of about 121 °C or more requires a shorter total time of sterilizing. On the other hand, a temperature T of about 118 °C or less, such as a temperature T of about 112 °C, during sterilizing requires a longer total period for sterilizing.

According to a preferred embodiment of the present disclosure, sterilizing is performed by sterile filtration.

According to a preferred embodiment of the present disclosure, the method for manufacturing the pharmaceutical composition according to the first aspect of the present disclosure does not comprise the step of sterilizing the container comprising the solution obtained from step d). However, it is understood that sterilizing is omitted only if at least one of steps a) to d) is carried out under aseptic conditions. In particular, it is preferable that step d) is carried out under aseptic conditions.

## The container

According to a fourth aspect, the present disclosure relates to a container comprising the pharmaceutical composition according to the first aspect of the present disclosure, wherein the container is a glass vial, a glass ampoule, a plastic ampoule, a plastic vial, a prefilled syringe, or an IV bag.

According to a preferred embodiment of the present disclosure, the container is a glass vial. According to another preferred embodiment of the present disclosure, the container is a glass ampoule. According to another preferred embodiment of the present disclosure, the container is a plastic ampoule. According to another preferred embodiment of the present disclosure, the container is a plastic vial. According to another preferred embodiment of the present disclosure, the container is a prefilled syringe. According to another preferred embodiment of the present disclosure, the container is an IV bag.

The container as used for the compositions of the present disclosure may be used according to the respective needs. While glass vials are more easy to handle at elevated temperatures, plastic ampules are not prone of breaking, or causing injuries to the medical staff. It is well within the skill of the skilled person to select the container accordingly. A difference in stability was not observed, and the choice of container has no influence on the results of stability of the pharmaceutical composition.

Definitions and general embodiments

As used herein, the terms "pharmaceutically acceptable salt of rocuronium" and "pharmaceutically acceptable rocuronium salt" are used interchangeably. As used herein, the term "rocuronium" refers to a cation of the name [3-hydroxy-10,13-dimethyl-2-morpholin-4-yl-16-(1-prop-2-enyl-2,3,4,5-tetrahydropyrrol-1-yl)-2,3,4,5,6,7,8,9,11,12,14,15,16,17-tetradecahydro-1H-cyclopenta[a]phenanthren-17-yl] acetate. In other words, the term "rocuronium" as used herein refers to a compound of the structure

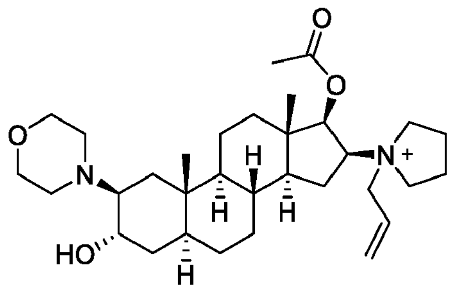

.

It is understood that rocuronium is a cation. Therefore, a pharmaceutically acceptable salt of rocuronium further comprises a pharmaceutically acceptable anion.

As used herein, the term "pharmaceutically acceptable anion" refers to an anion that is not toxic to mammals. Said pharmaceutically acceptable anion may have one, two, three, or more negative charges. Preferred pharmaceutically acceptable anions are $F^-$, $Cl^-$, $Br^-$, $I^-$, $OH^-$. A particularly preferred pharmaceutically acceptable anion is Br.

It is further understood that rocuronium is susceptible to degradation, especially when stored in aqueous formulations. A major degradation product is formed through acid catalyzed or base mediated hydrolysis of the acetic ester group to form a compound, which is also referred to as "impurity C" herein, of formula

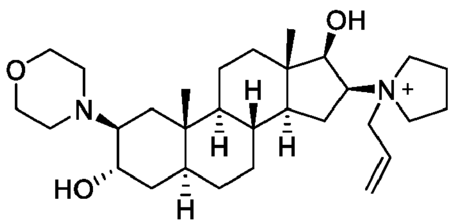

(Impurity C).

As used herein, the term "pharmaceutically acceptable cation" refers to a cation that is not toxic to mammals. Preferred pharmaceutically acceptable cations are lithium, sodium, potassium, aluminium, and zinc, cations made from organic bases such as choline, diethanolamine, morpholine, ammonium salts, quaternary salts such as tetramethylammonium salts.

As used herein, the term "sugar alcohol" refers to an organic compound, typically derived from sugars, containing one hydroxyl group (-OH) attached to each carbon atom. In other words, a "sugar alcohol" as used herein contains of from 3 to 12 carbon atoms, each of which contains one hydrogen atom. Alternatively, a sugar alcohol as used herein is a compound of formula I

$$HO\text{-}CH_2\text{-}[CH(OH)]_n\text{-}CH_2OH \qquad (I)$$

, wherein n is an integer of from 1 to 10, preferably of from 2 to 6, more preferably of from 3 to 5, even more preferably wherein n is 4.

As used herein, the term "monosaccharide", also called "simple sugar", refers to an organic compound that is the simplest form of sugar and the most basic units (monomers) from which all carbohydrates are built. Accordingly, the term "monosaccharide" refers to an organic compound of formula II

$$HO\text{-}CH_2\text{-}[CH(OH)]_n\text{-}(C{=}O)\text{-}[CH(OH)]_m\text{-}H \qquad (II)$$

, wherein n is an integer of from 1 to 10, preferably of from 2 to 6, more preferable wherein n is 3 or 4; and wherein m

is an integer of from 0 to 2, preferably wherein m is 0 or 1.

It is understood that a monosaccharide as used herein may be present in an open-chain form as shown in formula II, or as a lactol thereof, or as a mixture of an open-chain form as shown in formula II and a lactol thereof.

As used herein, the term "lactol" refers to the cyclic equivalent of a hemiacetal or a hemiketal. In other words, the compound is formed by the intramolecular nucleophilic addition of a hydroxyl group to the carbonyl group of an aldehyde or a ketone, such as an aldehyde or a ketone of formula II. It is understood that a lactol is often found as an equilibrium mixture with the corresponding hydroxyaldehyde or hydroxyketone of formula II. The equilibrium can favor either direction depending on ring size and other conformational effects.

Furthermore, it is understood that the monosaccharide as used herein is present in its D-configuration or in its L-configuration, or as a mixture of its D-configuration and its L-configuration.

The monosaccharides according to the present disclosure, can be classified into subgroups by virtue of the number of carbon atoms x contained in the monosaccharide. The number of carbons x contained in the monosaccharide is calculated as the sum of the integers n + m + 2 according to formula II. In other words, the number of carbon atoms x contained in the monosaccharide as used herein is calculated as

$$x = n + m + 2.$$

A monosaccharide, wherein x is 3, is referred to as "triose". A non-limiting example of triose is glyceraldehyde.

A monosaccharide, wherein x is 4, is referred to as "tetrose". Non-limiting examples of tetrose are erythrose, threose, and erythrulose.

A monosaccharide, wherein x is 5, is referred to as "pentose". Non-limiting examples of pentose are arabinose, lyxose, ribose, xylose, arabinose, ribulose, and xylolose.

A monosaccharide, wherein x is 6, is referred to as "hexose". Non-limiting examples of hexose are allose, altrose, glucose, mannose, gulose, idose, galactose, talose, psicose, fructose, sorbose, tagatose, and glutose.

A monosaccharide, wherein x is 7, is referred to as "heptose". Non-limiting examples of heptose are sedoheptulose, mannoheptulose, and *glycerol-manno*-heptose.

As used herein, the term "disaccharide" refers to an organic compound consisting of two monosaccharides which are joined by glycosidic linkage. Said glycosidic linkage may be $\alpha$-glycosidic, such as $\alpha(1\rightarrow2)$, $\alpha(1\rightarrow3)$, $\alpha(1\rightarrow4)$, $\alpha(1\rightarrow5)$, or $\alpha(1\rightarrow6)$; or $\beta$-glycosidic, such as $\beta(1\rightarrow2)$, $\beta(1\rightarrow3)$, $\beta(1\rightarrow4)$, or $\beta(1\rightarrow6)$.

Non-limiting examples of disaccharides are sucrose, lactose, maltose, trehalose, cellobiose, chitobiose, kojibiose, nigerose, isomaltose, sophorose, laminaribiose, gentiobiose, trehalulose, turanose, maltulose, leucrose, isomaltulose, gentiobiulose, mannobiose, melibiose, rutinose, rutinulose, and xylobiose.

As used herein, the term "polysaccharide" refers to an organic compound consisting of more than two monosaccharides which are joined by glycosidic linkage. Said glycosidic linkage may be $\alpha$-glycosidic, such as $\alpha(1\rightarrow2)$, $\alpha(1\rightarrow3)$, $\alpha(1\rightarrow4)$, $\alpha(1\rightarrow5)$, or $\alpha(1\rightarrow6)$; or $\beta$-glycosidic, such as $\beta(1\rightarrow2)$, $\beta(1\rightarrow3)$, $\beta(1\rightarrow4)$, or $\beta(1\rightarrow6)$. However, it is understood that a polysaccharide according to the present disclosure is soluble in cold water. Non-limiting examples of polysaccharides are pullulan, gum arabic, and dextrans.

As used herein, "HES" means hydroxyethyl starch.

As used herein, the osmolarity is determined according to European Pharmacopeia (Monograph Identifying Code 07/2019:20235 of Pharmacopoeia Europea 10.7).

As used herein, a "weak acid" is a substance that partially dissociates when it is dissolved water. In solution there is an equilibrium between the acid "HA" and the products of dissociation $H^+$ and $A^-$. As such, a weak acid has a $pK_a$ at 25 °C in the range of from 3 to 10. Non-limiting examples of weak acids are formic acid, acetic acid, citric acid, $NH4^+$, $H_2PO_4^-$, fumaric acid, $HCO_3^-$, $H_2CO_3$, $HSO_3^-$, maleic acid, malonic acid, HEPES, MES, TRIS, propionic acid, succinic acid, glycolic acid, gluconic acid, lactic acid, tartaric acid, glutamic acid, alkyl sulfonic acid, arylsulfonic acid, and the like.

As used herein, a "strong acid" is a substance "HA" that dissociates completely or substantially completely to its ions $H^+$ and $A^-$ when it is dissolved water. As such, a strong acid has a $pK_a$ at 25 °C of less than 3, preferably a strong acid as used herein has a $pK_a$ at 25 °C of -1 or less. Non-limiting examples of strong acids are HCl, HBr, HI, $H_2SO_4$, $H_2SO_3$, and $HNO_3$.

As used herein, a "strong base" is a compound of the structure XOH that dissociates completely or substantially completely to its ions $X^+$ and $OH^-$ when it is dissolved water, wherein X is a pharmaceutically acceptable cation. Non-limiting examples of strong bases are NaOH, LiOH, $Mg(OH)_2$, $Ca(OH)_2$, and KOH, preferably NaOH.

As used herein, the amount of rocuronium and impurity C is determined by according to European Pharmacopeia (Monograph Identifying Code 07/2017:1764 of Pharmacopoeia Europea 10.7).

As used herein, the term "room temperature" refers to a temperature of about 25 °C.

As used herein, the temperature T at which sterilization is performed is the temperature of the container.

As used herein, when sterilization is performed for a specific period, said period refers to the $F_0$ value. $F_0$ is a quantitative parameter used to evaluate the amount of heat transferred to a product or solution during a heat sterilization process, and describes the lethality performance of the process.

According to one embodiment, $F_0$ can be calculated through the following formula:

$$F_0 = \int_0^t 10^{\frac{T-121\,°C}{Z}} dt$$

wherein Z is 10 °C, and T is the temperature of the container at time point t. This formula can be applied when the temperature T of the container changes during sterilization.

In practice, the sterilizer, such as an autoclave or water cascade sterilizer, heats the product (e.g., container) for a certain period of time until the sterilization temperature of, e.g., 121 °C is reached. During the heating time for heating from 100 °C to 121 °C, and also during the cooling time, microorganisms are killed. Taking this killing effect during the heating and cooling time into account, the holding time can be shortened accordingly. Thus, the desired $F_0$ value can be achieved with a shorter overall process time.

In other words, the $F_0$ value of a terminal sterilization process is a measure for the lethality, expressed in terms of the equivalent time in minutes at a temperature T of 121 °C delivered by the process to the load in its container with reference to micro-organisms possessing a theoretical Z-value of 10 °C. For instance, an $F_0$ value of 1 min is the heat effect (lethal effect) of 121 °C within one minute.

According to another embodiment, the period $F_0$ for which sterilization is performed, is calculated on basis of the time t for which the container is held at a constant temperature T. If the container is held at a constant temperature T for a total time t during sterilization, the above formula simplifies as follows:

$$F_0 = t \cdot 10^{\frac{T-121\,°C}{Z}}$$

wherein Z is 10 °C, T is the temperature of the container, and t is the time for which the container is held at temperature T.

Furthermore, it the container is held at a temperature T of 112 °C for 15 min, the above equation simplifies to

$$F_0 = 15\,\text{min} \cdot 10^{\frac{112\,°C-121\,°C}{10\,°C}} = 15\,\text{min} \cdot 10^{-0.9} \approx 1.9\,\text{min}$$

In other words, a 15 min sterilization at 112 °C is equivalent, in terms of lethal effect, to about 1.9 min at 121 °C.

The term "about" in conjunction with a numerical value refers to normal deviations of said numerical value. It is to be understood that the term "about" can mean a deviation of ± 10 %, preferably ± 5 %, more preferably ± 2.5 % of said numeric value as indicated.

## Examples

### Example 1

To investigate the influence of pharmaceutical excipients on the stability of rocuronium in aqueous formulations, the inventors of the present disclosure prepared formulations by dissolving rocuronium bromide in water and dissolving the respective excipients to be tested in water and adjusting the pH using 20 % HCl to the final pH as indicated in Table 1 below. Then, the solution was filled into glass vials under air atmosphere which was sealed and sterilized at 121 °C for a period $F_0$ of at least 15 min.

In this way, compositions comprising rocuronium bromide at a concentration of 10 mg/mL, and the respective excipients (see Table 1 below) at a concentration of 25 mg/mL having the respective pH were obtained.

For stability studies, the vials were stored in the dark at 55 °C or 70 °C, respectively.

**Table 1** Overview on composition of test formulations for excipient screening

| Formulation | Excipient(s) | pH |
|---|---|---|
| **1A** | gluconolactone (25 mg/mL) + acetic acid (5 mg/mL) + citric acid (5 mg/mL) | 3.3 |
| **1B** | | 4.0 |
| **2B** | citric acid | 4.0 |
| **3B** | EDTA | 4.0 |
| **4A** | gluconic acid | 3.3 |
| **4B** | | 4.0 |
| **5B** | glucoronic acid | 4.0 |
| **6B** | lactobionic acid | 4.4 |
| **7B** | saccharic acid | 4.4 |
| **8A** | sorbitol | 3.3 |
| **8B** | | 4.0 |

After storing the formulations for several days, the amount of impurity C formed in the formulation was determined after sterilizing (day 0), after 8 days, and after 17 days of storage at 55 °C (Table 2) and 70 °C (Table 3), respectively.

**Table 2** Results of stability tests of excipient screening after storage at 55 °C

| Formulation | pH | Amount of impurity C[% (m/m)] after storage time [days] | | |
|---|---|---|---|---|
| | | 0 | 8 | 17 |
| **1A** | 3.3 | 0.7 | 1.2 | 2.1 |
| **4A** | | 0.7 | 1.1 | 1.7 |
| **8A** | | 0.6 | 0.9 | 1.4 |
| **1B** | 4 | 1.0 | 1.7 | 2.9 |
| **2B** | | 1.2 | 2.9 | 4.1 |
| **3B** | | 1.0 | 1.7 | 2.8 |
| **4B** | | 0.9 | 1.6 | 2.6 |
| **5B** | | 1.1 | 1.6 | 2.6 |
| **6B** | | 0.9 | 1.4 | 2.1 |
| **7B** | | 1.0 | 1.7 | 2.9 |
| **8B** | | 0.8 | 1.2 | 1.8 |

**Table 3** Results of stability tests of excipient screening after storage at 70 °C

| Formulation | pH | Amount of impurity C [% (m/m)] after storage time [days] | | |
|---|---|---|---|---|
| | | 0 | 8 | 17 |
| **1A** | 3.3 | 0.8 | 2.25 | 5.1 |
| **4A** | | 0.8 | 2.4 | 4.3 |
| **8A** | | 0.7 | 1.8 | 3.5 |

(continued)

| Formulation | pH | Amount of impurity C [% (m/m)] after storage time [days] | | |
|---|---|---|---|---|
| | | 0 | 8 | 17 |
| **1B** | 4 | 1.0 | 3.5 | 7.2 |
| **2B** | | 1.0 | 6.5 | 11.9 |
| **3B** | | 1.0 | 3.8 | 7.0 |
| **4B** | | 1.0 | 3.5 | 6.8 |
| **5B** | | 1.0 | 3.8 | 12.8 |
| **6B** | | 1.0 | 2.3 | 5.3 |
| **7B** | | 1.0 | 3.5 | 7.9 |
| **8B** | | 1.0 | 2.1 | 4.1 |

From the stability studies, it can be concluded that excipients containing a carboxylic acid group, such as compositions 1A/B, 2A/B, 3A/B, 4A/4B, 5A/B, 6A/B, and 7A/B showed reduced stability of rocuronium after storage. However, a composition comprising sorbitol, i.e. composition 8A/B, shows improved stability of rocuronium during storage, both at pH 3.3 and pH 4.

Without wishing to be bound by theory, it may be assumed that anionic agents with chelating properties are detrimental to the stability of rocuronium in aqueous formulations due to interactions of the anionic moieties with the cationic quaternary ammonium salt of rocuronium bromide.

However, the best stability results were found for the neutral polyol sorbitol showing the best stabilizing effect. This finding goes contrary to the knowledge obtained from prior art formulations which taught a beneficial effect of organic anions on stability (e.g. acetate, citrate and/or gluconate anions). The finding of the present inventors is thus surprising.

Example 2

To further investigate the stabilizing effects of polyols on the stability and shelf-life of pharmaceutical compositions of rocuronium, the inventors of the present disclosure prepared further formulations by dissolving rocuronium bromide in water and adjusting the pH using HCl, dissolving the respective excipients to be tested in water and adjusting the pH using HCl, combining said solutions of rocuronium bromide and the excipient and adjusting the pH of said combined solution with HCl or NaOH to pH 4.0. Then, the solution was filled into glass vials under air atmosphere which was sealed and sterilized at 112 °C for a period $F_0$ of 8 min or 121 °C for a period $F_0$ of 20 min, respectively.

In this way, compositions comprising rocuronium bromide at a concentration of 10 mg/mL were obtained. The concentration of the excipient was as indicated for the respective formulation in Table 4. For stability studies, the vials were stored in the dark at 20 °C, 30 °C or 40 °C, respectively.

**Table 4** Overview on composition of test formulations for excipient screening

| Formulation | Glucono-lactone [mg/mL] | Na citrate 2 $H_2O$ [mg/mL] | Na acetate ▪ 3 $H_2O$ [mg/mL] | Lacto-bionic acid [mg/mL] | sorbitol [mg/mL] |
|---|---|---|---|---|---|
| **1B** | 25.0 | 5.0 | 5.0 | - | - |
| **6B** | - | - | - | 25.0 | - |
| **6C** | 25.0 | - | - | 25.0 | - |
| **6D** | 25.0 | 5.0 | - | 25.0 | - |
| **6E** | 25.0 | 5.0 | 5.0 | 25.0 | - |
| **6F** | - | 5.0 | 5.0 | 25.0 | - |
| **6G** | - | - | - | 5.0 | - |
| **6H** | - | - | - | 11.1 | - |
| **6I** | 13.9 | 2.8 | 2.8 | 11.1 | - |

(continued)

| Formulation | Glucono-lactone [mg/mL] | Na citrate 2 $H_2O$ [mg/mL] | Na acetate ▪ 3 $H_2O$ [mg/mL] | Lacto-bionic acid [mg/mL] | sorbitol [mg/mL] |
|---|---|---|---|---|---|
| **8B** | - | - | - | - | 25.0 |
| **8C** | 25.0 | - | - | - | 25.0 |
| **8D** | 25.0 | 5.0 | - | - | 25.0 |
| **8E** | 25.0 | 5.0 | 5.0 | - | 25.0 |
| **8F** | - | 5.0 | 5.0 | - | 25.0 |
| **8G** | - | - | - | - | 5.0 |
| **8H** | - | - | - | - | 11.1 |
| **8I** | 13.9 | 2.8 | 2.8 | - | 11.1 |
| **9** | - | - | - | 12.5 | 12.5 |

Firstly, the influence of the different stabilizing agents on the formation of impurity C upon sterilizing under different conditions was investigated and summarized in Table 5 below.

**Table 5** Formation of impurity C during sterilization

| formulation | Amount of impurity C [% (m/m)] | | |
|---|---|---|---|
| **sterilizing** | **before** | **$F_0$ of 8 min @ 112 °C** | **$F_0$ of 20 min @ 121 °C** |
| **1B** | 0.50 | 1.0 | 0.87 |
| **6B** | 0.39 | 0.75 | 0.65 |
| **6C** | 0.60 | 1.1 | 0.94 |
| **6D** | 0.56 | 1.1 | 0.95 |
| **6E** | 0.50 | 1.1 | 0.93 |
| **6F** | 0.40 | 0.86 | 0.73 |
| **6G** | 0.33 | 0.65 | - |
| **6H** | 0.36 | 0.67 | - |
| **6I** | 0.44 | 0.91 | - |
| **8B** | 0.34 | 0.55 | 0.49 |
| **8C** | 0.69 | 1.1 | 1.0 |
| **8D** | 0.62 | 1.1 | 0.99 |
| **8E** | 0.55 | 1.1 | 0.93 |
| **8F** | 0.33 | 0.79 | 0.67 |
| **8G** | 0.32 | 0.56 | - |
| **8H** | 0.32 | 0.57 | - |
| **8I** | 0.41 | 0.85 | - |
| **9** | 0.34 | 0.66 | - |
| -: not determined | | | |

It can be concluded from the results of the investigations on the formation of impurity C during sterilization that the presence of carboxylic acids and derivatives thereof, such as acetic acid, citric acid, and/or gluconolactone, reduce

the stability of rocuronium during sterilization. However, the presence of sorbitol as stabilizer improves the stability of rocuronium during sterilization. Even more, in the example a concentration-dependent stabilizing effect of sorbitol was observed (c.f. 8G vs. 8H vs. 8B). Interestingly, the opposite is the case for Lactobionic acid. Without wishing to by bound to theory, two effects conclude from the use of Lactobionic acid. On the one hand, a stabilizing effect of the sugar part of the molecule. On the other hand, the detrimental effect of the carboxylic acid, as it is even more dominant in gluconolactone / gluconic acid.

Furthermore, the inventors of the present disclosure investigated into the storage stability of the rocuronium formulations. After storing the formulations for three months at 25 °C, 30 °C, or 40 °C, respectively, the amount of impurity C formed in the formulation was determined.

**Table 6** Results of stability tests of excipient screening after storage for 3 months

| formulation | Amount of impurity C [% (m/m)] after | | | | | |
|---|---|---|---|---|---|---|
| | sterilizing | 3 months at | | | sterilizing | 3 months at |
| | | 25 °C | 30 °C | 40 °C | | 40 °C |
| $F_0$ of | 8 min | 8 min | 8 min | 8 min | 20 min | 20 min |
| T | 112 °C | 112 °C | 112 °C | 112 °C | 121 °C | 121 °C |
| 1B | 1.0 | 1.5 | 1.9 | 3.6 | 0.87 | 3.5 |
| 6B | 0.75 | 1.1 | 1.4 | 2.5 | 0.65 | 2.4 |
| 6C | 1.1 | 1.6 | 1.9 | 3.4 | 0.94 | 3.3 |
| 6D | 1.1 | 1.6 | 1.9 | 3.6 | 0.95 | 3.4 |
| 6E | 1.1 | 1.6 | 2.0 | 3.8 | 0.93 | 3.7 |
| 6F | 0.86 | 1.4 | 1.7 | 3.2 | 0.73 | 3.7 |
| 6G | 0.65 | - | - | 2.0 | - | - |
| 6H | 0.67 | - | - | 2.2 | - | - |
| 6I | 0.91 | - | - | 3.1 | - | - |
| 8B | 0.55 | 0.85 | 1.0 | 1.8 | 0.49 | 1.6 |
| 8C | 1.1 | 1.5 | 1.8 | 3.1 | 1.0 | 3.0 |
| 8D | 1.1 | 1.6 | 1.9 | 3.4 | 0.99 | 3.3 |
| 8E | 1.1 | 1.6 | 2.0 | 3.6 | 0.93 | 3.5 |
| 8F | 0.79 | 1.3 | 1.6 | 3.0 | 0.67 | 2.9 |
| 8G | 0.56 | - | - | 1.7 | - | - |
| 8H | 0.57 | - | - | 1.7 | - | - |
| 8I | 0.85 | - | - | - | - | - |
| 9 | 0.66 | - | - | 2.1 | | |
| -: not determined | | | | | | |

Also the results of the stability studies confirm that the presence of carboxylic acids and derivatives thereof, such as acetic acid, citric acid, and/or gluconolactone, reduces the stability of rocuronium in an aqueous formulation.

Furthermore, it is confirmed that sorbitol even at a low concentration of 5 mg/mL stabilizes rocuronium, during sterilization and thereafter.

Example 3

To further confirm the stabilizing effects of polyols as opposed to carboxylic acids or carboxylic acid esters (e.g. gluconolactone), the inventors of the present disclosure prepared further formulations according to the procedure of example 1 and adjusting the pH of said combined solution with HCl or NaOH to pH 3.0 or pH 4.0, respectively. Then,

the solution was filled into glass vials under air atmosphere which was sealed and sterilized at 121 °C for a period of $F_0$ of at least 15 min.

In this way, compositions comprising rocuronium bromide at a concentration of 10 mg/mL, and at a concentration of the respective stabilizing agent as indicated in Table 7 below, were obtained.

**Table 7** Overview on composition of test formulations for excipient screening

| Formulation | stabilizer | concentration [mg/mL] | tonicity agent | concentration [mg/mL] | pH adjusted to |
|---|---|---|---|---|---|
| **10A** | glucose | 45.0 | - | - | 3.0 |
| **10B** | glucose | 45.0 | - | - | 4.0 |
| **11A** | sorbitol | 45.0 | - | - | 3.0 |
| **11B** | sorbitol | 45.0 | - | - | 4.0 |
| **12A** | sucrose | 90.0 | - | - | 3.0 |
| **12B** | sucrose | 90.0 | - | - | 4.0 |
| **13A** | xylitol | 45.0 | - | - | 3.0 |
| **13B** | xylitol | 45.0 | - | - | 4.0 |
| **14A** | glucose | 22.5 | NaCl | 4.5 | 3.0 |
| **14B** | glucose | 22.5 | NaCl | 4.5 | 4.0 |
| **15A** | sorbitol | 22.5 | NaCl | 4.5 | 3.0 |
| **15B** | sorbitol | 22.5 | NaCl | 4.5 | 4.0 |
| **16** | mannitol | 45.0 | - | - | 4.0 |
| **17** | lactose | 45.0 | - | - | 4.0 |
| **18** | trehalose | 90.0 | - | - | 4.0 |
| **19** | HES130 | 45.0 | - | - | 4.0 |
| **20** | glycerol | 22.5 | - | - | 4.0 |

Furthermore, the storage stability of the rocuronium formulations were investigated. After storing the formulations for 17 days at 50 °C, or 70 °C, respectively, the amount of impurity C formed in the formulation was determined.

**Table 8** Results of stability tests of excipient screening after storage for 17 days.

| formulation | Amount of impurity C [% (m/m)] at day 0 | Amount of impurity C [% (m/m)] after 17 days at | |
|---|---|---|---|
| | | **50** °C | **70** °C |
| **10A** | 0.65 | 1.05 | 2.70 |
| **10B** | 0.51 | 1.10 | 3.39 |
| **11A** | 0.60 | 0.97 | 2.63 |
| **11B** | 0.55 | 1.14 | 3.48 |
| **12A** | 0.65 | 0.96 | 2.59 |
| **12B** | 0.51 | 1.11 | 3.56 |
| **13A** | 0.71 | 1.02 | 2.37 |
| **13B** | 0.57 | 1.33 | 3.58 |
| **14A** | 0.62 | 0.94 | - |
| **14B** | 0.51 | 1.17 | - |
| **15A** | 0.67 | 0.94 | - |
| **15B** | 0.54 | 1.19 | - |

(continued)

| formulation | Amount of impurity C [% (m/m)] at day 0 | Amount of impurity C [% (m/m)] after 17 days at | |
|---|---|---|---|
| | | **50** °C | **70** °C |
| **16** | 0.54 | 1.08 | 3.48 |
| **17** | 0.56 | 1.29 | 3.78 |
| **18** | 0.54 | 1.21 | 3.51 |
| **19** | 0.52 | 1.14 | 3.71 |
| **20** | 0.54 | 1.13 | 3.75 |
| -: not determined | | | |

Also the results of the further stability studies confirm that the presence of polyols, such as glucose, sorbitol, xylitol, lactose, trehalose, HES, and glycerol stabilizes formulations of rocuronium in that only very little amount of impurity C is formed during storage under harsh conditions, such as at 70 °C. Said effect can be observed over a wide range of pH values.

Of note, all compositions show considerably less than 2 % of formation of impurity C and can, therefore, be considered as stable.

Additionally, it is noted that the presence of NaCl as tonicity agent does not significantly change the stability of rocuronium in the formulations.

Example 4

To investigate the stability of the novel rocuronium formulation compared to the current state of the art, the commercially available marketed US product "Rocuronium Bromide Injection 50 mg/5 mL" by XGen Pharmaceuticals DJB, Inc. (herein referred to as formulation 21), which is prepared aseptically without heat sterilization, was used as a benchmark. According to its product specifications, said product must to be stored in refrigerated form to achieve acceptable levels of impurity C and resultantly acceptable stability for a shelf-life of 24-months. Freshly produced "Rocuronium Bromide Injection 50 mg/5 mL" with a remaining shelf-life over 23.5 months was investigated in a stability study in a head-to-head comparison with the novel formulation 8B of rocuronium bromide as prepared according to example 2.

The compositions were stored at 25 °C for 3 months and the increase of impurity C was investigated.

**Table 9** Results of stability tests.

| Formulation | stabilizer | stabilizer concentration [mg/mL] | tonicity agent | pH adjusted to | Increase of impurity C after 3 months @ 25°C | Increase of impurity C / month @ 25 °C |
|---|---|---|---|---|---|---|
| **21** | Sodium acetate | 2.0 | NaCl | 4.0 with acetic acid and/or NaOH | 0.63 | 0.21 |
| **8B** | Sorbitol | 25.0 | - | 4.0 | 0.30 | 0.10 |

In line with the previous examples, the direct comparison of the state-of-the-art composition 21 with the novel formulation 8B demonstrates the superiority in stability of polyol-comprising rocuronium compositions, which shows less than 50 % of degradation over 3 months at 25 °C.

## Embodiments

1. A pharmaceutical composition comprising

a) a pharmaceutically acceptable salt of rocuronium,
b) at least one stabilizer,

c) optionally a tonicity agent, and
d) water;

wherein the at least one stabilizer is a polyol, and wherein the polyol does not comprise a -COOX group, wherein X is hydrogen or a pharmaceutically acceptable cation.

2. The pharmaceutical composition according to embodiment 1, wherein the polyol is a sugar alcohol, or a monosaccharide, or a disaccharide, or a polysaccharide.

3. The pharmaceutical composition cording to embodiment 1 or 2, wherein the polyol is a sugar alcohol of the formula I

$$HO\text{-}CH_2\text{-}[CH(OH)]_{n\text{-}C}H_2OH \qquad (I)$$

, wherein n is an integer of from 1 to 10, preferably of from 2 to 6, more preferably of from 3 to 5, even more preferably wherein n is 4.

4. The pharmaceutical composition according to any one of embodiments 1 to 3, wherein the at least one stabilizer is mannitol, glycerol or sorbitol.

5. The pharmaceutical composition according to embodiment 1 or 2, wherein the polyol is a monosaccharide of the formula II

$$HO\text{-}CH_2\text{-}[CH(OH)]_n\text{-}(C{=}O)\text{-}[CH(OH)]_m\text{-}H \qquad (II)$$

,

or an intramolecular lactole thereof, wherein
n is an integer of from 1 to 10, preferably of from 2 to 6, more preferable wherein n is 3 or 4; and wherein
m is an integer of from 0 to 2, preferably wherein m is 0 or 1.

6. The pharmaceutical composition according to any one of embodiments 1, 2, or 5, wherein the polyol is selected from the group consisting of glucose, fructose, galactose, and mixtures thereof.

7. The pharmaceutical composition according to embodiment 1 or 2, wherein the polyol is a disaccharide selected from the group consisting of sucrose, maltose, maltulose, isomaltose, lactose, lactulose, trehalose, and mixtures thereof.

8. The pharmaceutical composition according to any one of embodiments 1 to 7, wherein the pharmaceutical composition has a pH in the range of from about 3.0 to about 4.5, preferably of from about 3.2 to about 4.2, or of from about 3.0 to about 3.5, more preferably of from about 3.8 to about 4.2.

9. The pharmaceutical composition according to any one of embodiments 1 to 8, wherein the pharmaceutical composition comprises the pharmaceutically acceptable salt of rocuronium in a concentration of from about 0.1 mg/mL to about 100 mg/mL, preferably of from about 1 mg/mL to about 20 mg/mL, more preferably of from about 5 mg/mL to about 15 mg/mL, even more preferably of from about 8 mg/mL to about 12 mg/mL, most preferably about 10 mg/mL.

10. The pharmaceutical composition according to any one of embodiments 1 to 9, wherein the pharmaceutically acceptable salt of rocuronium is rocuronium bromide (CAS No. 119302-91-9).

11. The pharmaceutical composition according to any one of embodiments 1 to 10, wherein the pharmaceutical composition has an osmolarity of 600 mOsmol/L or less, preferably wherein the pharmaceutical composition has an osmolarity in the range of from about 32 mOsmol/L to about 600 mOsmol/L, more preferably in the range of from about 100 mOsmol/L to about 600 mOsmol/L, more preferably of from about 120 mOsmol/L to about 500 mOsmol/L, more preferably of from about 150 mOsmol/L to about 450 mOsmol/L, more preferably of from about 200 mOsmol/L to about 400 mOsmol/L, more preferably of from about 230 mOsmol/L to about 380 mOsmol/L, more preferably of from about 250 mOsmol/L to about 350 mOsmol/L, most preferably of from about 270 mOsmol/L to about 310 mOsmol/L.

12. The pharmaceutical composition according to any one of embodiments 1 to 11, wherein the pharmaceutical composition does not contain EDTA, or glycine, or citric acid, or acetic acid, or gluconic acid, or a salt thereof, or a derivative thereof, preferably wherein the pharmaceutical composition does not contain a weak acid or a salt thereof or a derivative thereof.

13. The pharmaceutical composition according to any one of embodiments 1 to 12, wherein the pharmaceutical composition has a titratable acidity of about 100 mmol/mL or less, preferably about 80 mmol/mL or less, more preferably about 60 mmol/mL, more preferably about 40 mmol/mL or less, most preferably about 20 mmol/mL or less.

14. A method for manufacturing the pharmaceutical composition according to any one of embodiments 1 to 13 comprising the steps of

a) dissolving the pharmaceutically acceptable salt of rocuronium in water and adjusting the pH with a strong acid to provide a solution I;
b) dissolving the at least one stabilizer in water and adjusting the pH with a strong acid to provide a solution II;
c) mixing said solution I and said solution II to provide a solution III and optionally adjusting the pH with a strong acid or a strong base;
d) filling said solution III into a container and sealing said container; and
e) optionally sterilizing said container comprising solution III.

15. A method for manufacturing the pharmaceutical composition according to any one of embodiments 1 to 13 comprising the steps of

a) dissolving the at least one stabilizer in water;
b) dissolving the pharmaceutically acceptable salt of rocuronium in said solution and optionally adjusting the pH with a strong acid or strong base;
c) filtering the solution;
d) filling said solution into a container and sealing said container; and
e) optionally sterilizing said container comprising the solution.

16. The method according to embodiment 14 or 15, wherein sterilizing is performed for a period $F_0$ of at least about 1 min, preferably at least about 5 min, more preferably at least about 8 min.

17. The method according to embodiment 14 or 16, wherein sterilizing is performed for a period $F_0$ of about 12 h or less, preferably of about 1 h or less, preferably for a period $F_0$ of about 45 min or less, more preferably of about 30 min or less, most preferably of about 20 min or less.

18. The method according to any one of embodiments 14 to 17, wherein sterilizing is performed at a temperature T of about 121 °C or more, preferably at a temperature T of about 121 °C.

19. The method according to any one of embodiments 14 to 18, wherein sterilizing is performed at a temperature T of about 118 °C or less, preferably at a temperature T of about 112 °C.

20. A container comprising the pharmaceutical composition according to any one of embodiments 1 to 13, wherein the container is a glass vial, a glass ampoule, a plastic ampoule, a plastic vial, a prefilled syringe, or an IV bag.

## Claims

**1.** A pharmaceutical composition comprising

a) a pharmaceutically acceptable salt of rocuronium,
b) at least one stabilizer,
c) optionally a tonicity agent, and
d) water;

wherein the at least one stabilizer is a polyol, and wherein the polyol does not comprise a -COOX group, wherein X is hydrogen or a pharmaceutically acceptable cation.

**2.** The pharmaceutical composition according to claim 1, wherein the polyol is a sugar alcohol, or a monosaccharide, or a disaccharide, or a polysaccharide.

**3.** The pharmaceutical composition cording to claim 1 or 2, wherein the polyol is a sugar alcohol of the formula I

$$HO\text{-}CH_2\text{-}[CH(OH)]_n\text{-}CH_2OH \qquad (I)$$

, wherein n is an integer of from 1 to 10, preferably of from 2 to 6, more preferably of from 3 to 5, even more preferably wherein n is 4, most preferably wherein the at least one stabilizer is mannitol, glycerol or sorbitol.

**4.** The pharmaceutical composition according to claim 1 or 2, wherein the polyol is a monosaccharide of the formula II

$$HO\text{-}CH_2\text{-}[CH(OH)]_n\text{-}(C{=}O)\text{-}[CH(OH)]_m\text{-}H \qquad (II)$$

,

or an intramolecular lactole thereof, wherein
n is an integer of from 1 to 10, preferably of from 2 to 6, more preferable wherein n is 3 or 4; and wherein
m is an integer of from 0 to 2, preferably wherein m is 0 or 1, most preferably
wherein the polyol is selected from the group consisting of glucose, fructose, galactose, and mixtures thereof.

**5.** The pharmaceutical composition according to claim 1 or 2, wherein the polyol is a disaccharide selected from the group consisting of sucrose, maltose, maltulose, isomaltose, lactose, lactulose, trehalose, and mixtures thereof.

**6.** The pharmaceutical composition according to any one of claims 1 to 5, wherein the pharmaceutical composition has a pH in the range of from about 3.0 to about 4.5, preferably of from about 3.2 to about 4.2, or of from about 3.0 to about 3.5, more preferably of from about 3.8 to about 4.2.

**7.** The pharmaceutical composition according to any one of claims 1 to 6, wherein the pharmaceutical composition comprises the pharmaceutically acceptable salt of rocuronium in a concentration of from about 0.1 mg/mL to about 100 mg/mL, preferably of from about 1 mg/mL to about 20 mg/mL, more preferably of from about 5 mg/mL to about 15 mg/mL, even more preferably of from about 8 mg/mL to about 12 mg/mL, most preferably about 10 mg/mL.

**8.** The pharmaceutical composition according to any one of claims 1 to 7, wherein the pharmaceutically acceptable salt of rocuronium is rocuronium bromide (CAS No. 119302-91-9).

**9.** The pharmaceutical composition according to any one of claims 1 to 8, wherein the pharmaceutical composition has an osmolarity of 600 mOsmol/L or less, preferably wherein the pharmaceutical composition has an osmolarity in the range of from about 32 mOsmol/L to about 600 mOsmol/L, more preferably in the range of from about 100 mOsmol/L to about 600 mOsmol/L, more preferably of from about 120 mOsmol/L to about 500 mOsmol/L, more preferably of from about 150 mOsmol/L to about 450 mOsmol/L, more preferably of from about 200 mOsmol/L to about 400 mOsmol/L, more preferably of from about 230 mOsmol/L to about 380 mOsmol/L, more preferably of from about 250 mOsmol/L to about 350 mOsmol/L, most preferably of from about 270 mOsmol/L to about 310 mOsmol/L.

**10.** The pharmaceutical composition according to any one of claims 1 to 9, wherein the pharmaceutical composition does not contain EDTA, or glycine, or citric acid, or acetic acid, or gluconic acid, or a salt thereof, or a derivative thereof, preferably wherein the pharmaceutical composition does not contain a weak acid or a salt thereof or a derivative thereof.

**11.** The pharmaceutical composition according to any one of claims 1 to 10, wherein the pharmaceutical composition has a titratable acidity of about 100 mmol/mL or less, preferably about 80 mmol/mL or less, more preferably about 60 mmol/mL, more preferably about 40 mmol/mL or less, most preferably about 20 mmol/mL or less.

**12.** A method for manufacturing the pharmaceutical composition according to any one of claims 1 to 11 comprising the steps of

a) dissolving the pharmaceutically acceptable salt of rocuronium in water and adjusting the pH with a strong

acid to provide a solution I;
b) dissolving the at least one stabilizer in water and adjusting the pH with a strong acid to provide a solution II;
c) mixing said solution I and said solution II to provide a solution III and optionally adjusting the pH with a strong acid or a strong base;
d) filling said solution III into a container and sealing said container; and
e) optionally sterilizing said container comprising solution III.

**13.** A method for manufacturing the pharmaceutical composition according to any one of claims 1 to 11 comprising the steps of

a) dissolving the at least one stabilizer in water;
b) dissolving the pharmaceutically acceptable salt of rocuronium in said solution and optionally adjusting the pH with a strong acid or strong base;
c) filtering the solution;
d) filling said solution into a container and sealing said container; and
e) optionally sterilizing said container comprising the solution.

**14.** The method according to any one of claim 12 or 13, wherein sterilizing is performed

a) at a temperature T of about 121 °C or more, preferably at a temperature T of about 121 °C; or
b) at a temperature T of about 118 °C or less, preferably at a temperature T of about 112 °C.

**15.** A container comprising the pharmaceutical composition according to any one of claims 1 to 11, wherein the container is a glass vial, a glass ampoule, a plastic ampoule, a plastic vial, a prefilled syringe, or an IV bag.

Europäisches Patentamt
European Patent Office
Office européen des brevets

# EUROPEAN SEARCH REPORT

Application Number
EP 22 20 7767

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | CN 105 496 954 A (LI JIE) 20 April 2016 (2016-04-20)<br>* the whole document *<br>----- | 1-3,8, 13-15<br>5,10-12 | INV.<br>A61K9/00<br>A61K9/08<br>A61K47/26<br>A61K31/00<br>A61K47/10 |
| X<br>Y | CN 111 374 942 A (SHANGDONG NEW TIME PHARM PROD) 7 July 2020 (2020-07-07)<br>* the whole document *<br>----- | 1,6,8,9<br>5,10-12 | |
| X<br>Y | EP 2 712 611 A1 (BRAUN MELSUNGEN AG [DE]) 2 April 2014 (2014-04-02)<br>* the whole document *<br>----- | 1-4,6-8<br>5,10-12 | |

TECHNICAL FIELDS SEARCHED (IPC)

A61K

The present search report has been drawn up for all claims

2

| Place of search | Date of completion of the search | Examiner |
|---|---|---|
| The Hague | 26 April 2023 | Raposo, Antonio |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO.

EP 22 20 7767

This annex lists the patent family members relating to the patent documents cited in the above-mentioned European search report. The members are as contained in the European Patent Office EDP file on
The European Patent Office is in no way liable for these particulars which are merely given for the purpose of information.

26-04-2023

| Patent document cited in search report | Publication date | Patent family member(s) | Publication date |
|---|---|---|---|
| CN 105496954 A | 20-04-2016 | NONE | |
| CN 111374942 A | 07-07-2020 | NONE | |
| EP 2712611 A1 | 02-04-2014 | AU 2013322897 A1 | 29-01-2015 |
| | | BR 112015000688 A2 | 27-06-2017 |
| | | CA 2878714 A1 | 03-04-2014 |
| | | CN 104519872 A | 15-04-2015 |
| | | CY 1119086 T1 | 10-01-2018 |
| | | EP 2712611 A1 | 02-04-2014 |
| | | EP 2900216 A1 | 05-08-2015 |
| | | ES 2628076 T3 | 01-08-2017 |
| | | HK 1205694 A1 | 24-12-2015 |
| | | IL 236042 A | 29-06-2017 |
| | | JP 6347784 B2 | 27-06-2018 |
| | | JP 2015531359 A | 02-11-2015 |
| | | KR 20150058259 A | 28-05-2015 |
| | | MX 356212 B | 18-05-2018 |
| | | PL 2900216 T3 | 31-10-2017 |
| | | PT 2900216 T | 23-06-2017 |
| | | RU 2015115430 A | 20-11-2016 |
| | | TW 201412317 A | 01-04-2014 |
| | | US 2015216979 A1 | 06-08-2015 |
| | | US 2020000925 A1 | 02-01-2020 |
| | | WO 2014048836 A1 | 03-04-2014 |
| | | ZA 201408945 B | 25-11-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *CHEMICAL ABSTRACTS,* 119302-91-9